(19) Europäisches Patentamt / European Patent Office / Office européen des brevets

(11) **EP 2 609 892 B1**

(12) **EUROPEAN PATENT SPECIFICATION**

(45) Date of publication and mention
of the grant of the patent:
**08.08.2018 Bulletin 2018/32**

(51) Int Cl.:
***A61F 2/04*** *(2013.01)*  ***A61M 27/00*** *(2006.01)*
***A61F 2/88*** *(2006.01)*

(21) Application number: **12198620.2**

(22) Date of filing: **20.12.2012**

(54) **Ureteral stent**

Harnröhrenstent

Stent urétéral

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.12.2011 US 201161580935 P**

(43) Date of publication of application:
**03.07.2013 Bulletin 2013/27**

(73) Proprietor: **Cook Medical Technologies LLC
Bloomington, IN 47404 (US)**

(72) Inventor: **Devereux, Paul David
Dublin 18 (IE)**

(74) Representative: **Georgiou, Sarah Caroline
Williams Powell
Staple Court
11 Staple Inn Buildings
London WC1V 7QH (GB)**

(56) References cited:
**WO-A1-01/91668    US-A1- 2004 087 886**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

RELATED APPLICATIONS

TECHNICAL FIELD

The technical field of this invention is implantable medical devices, and in particular a stent useful for urinary drainage.

BACKGROUND

Indwelling ureteral stents have been widely used for years. Such stents are placed in the ureter, which is the duct between the kidney and the bladder, for the purpose of establishing and/or maintaining an open, patent flow of urine from the kidney to the bladder.

Ureteral stents may be used to retain patency of the ureteral lumen and to continue normal urinary drainage following the treatment and removal of stones and calculi from kidneys and ureters. To treat this condition, several individual steps are involved. In one procedure, these steps include placing a relatively narrow guidewire through a urethra and a bladder, and then through the ureter and into the kidney. After the guidewire is placed, a catheter is run along the guidewire, dilating the body passage (the urethra and the ureter) as it moves down the guidewire. The access sheath also dilates the body passages as it moves from outside the body, through the urethra, and into the ureter, down the desired location, and into or very near the kidney.

The physician may then remove calculi and stones through the access sheath, using a grasper, a retrieval basket or other device. The access sheath protects the ureter from repeated passage of the retrieval device while the stones or calculi are removed. After the stones are removed, the ureteral stent may be placed into the ureter through the access sheath, using the catheter or a pushing tube to position the stent.

The typical ureteral stent can be composed of various radiopaque polymers, including polyethylene, silicone, polyurethane, and thermoplastic elastomer. These stents are retained in the ureter by a retentive anchoring portion, such as a curved shape, pigtail, coil, J-shape, or hook configuration, at either end of the stent that engages the walls of the bladder and the kidney, respectively. The stent is resilient to allow it to be straightened for insertion into a body passageway and returned to its predetermined retentive anchoring shape when in situ. There can be problems, however, with ureteral stents, as urine may fail to drain through the stent. This may be due to a number of reasons, such as extrinsic compression of the stent or blockage of the drainage mechanism of the stent by encrustation. Furthermore, there can be problems associated with migration of the urethral stent from the original implantation site either upward into the kidney of the patient or downward into the bladder of the patient.

WO 01/91668 and US2004/0087886 describe ureteral stents for draining fluid from a patient's kidney into their bladder. In particular, WO 01/91668 describes a ureteral stent which includes an elongate portion extending within the ureter from the kidney to the bladder. At one end of the elongate portion, a retention portion retains the stent within the kidney, and at the other end of the elongate portion a flared portion is positioned in the bladder.

BRIEF SUMMARY

The present invention is defined in the appended claims.

The present disclosure relates to a stent for placing in a body passage of a patient. The stent has an elongated portion having a proximal end and a distal end. The elongated portion defines a lumen throughout the stent. The stent has a distal anchoring member on the distal end of the elongated portion. The stent may also have a proximal anchoring member on the proximal end of the elongated portion. The anchoring member comprises a wall curving outward from each end of the elongated portion of the stent.

The wall of the anchoring member includes a plurality of support struts. In one example, the plurality of support struts is integral with the wall of the anchoring member. The plurality of support struts may be comprised of shape memory materials. In another aspect, the wall of the anchoring member has a first opening having a first diameter and a second opening having a second diameter, forming an edge. The second diameter of the second opening is greater than the first diameter of the first opening. The wall of the anchoring member may be symmetrical along a longitudinal axis.

In another aspect of the present disclosure, an anchoring member for a stent is provided. The anchoring member may be a drainage device. The drainage device includes a wall which may have a generally concave configuration. The wall includes a first opening having a first diameter and a second opening having a second diameter defining an edge. The drainage device further includes a plurality of support struts disposed about an outer surface of the wall. The second diameter of the second opening of the wall is greater than the first diameter of the first opening of the wall. The wall of the drainage and anchoring member may be comprised of material selected from the group consisting of polyesters, fluorinated polymers, and polyurethanes. The plurality of support struts are disposed longitudinally about the circumference of the wall. The plurality of support struts may be integral with the outer surface of the wall. The plurality of support struts may be comprised of a shape memory material.

In another aspect of the present disclosure a stent for placing in a body passage of a patient is provided. The stent includes a coiled wire. The coiled wire has an internal lumen. The internal lumen is configured to communicate outside of the coiled wire through small spaces between adjacent coils. The stent has an anchoring member on a distal end of the coiled wire. The anchoring member includes a wall curving outward from each end of the coiled wire. A plurality of support struts is disposed longitudinally about the circumference of the wall. In one aspect, the wire is selected from the group consisting of MP35N, MP159, Astroloy M, Inconel 625, 315 stainless steel, 35N LT, Bidur 108, titanium, and Hastelloy S.

BRIEF DESCRIPTION OF THE DRAWINGS

Embodiments of the present invention are described below, by way of example only, with reference to the accompanying drawings, in which:

FIG. 1 depicts an embodiment of a ureteral stent of the present invention.
FIG. 2. depicts a side view of the embodiment of the ureteral stent of FIG. 1.
FIG. 3. depicts a top view of an end of the embodiment of the ureteral stent of FIG. 1.
FIG. 4 depicts a technique for ureteral stent placement.
FIG. 5 depicts a sheath and catheter useful for placing a ureteral stent.
FIG. 6 depicts an embodiment of a ureteral stent having an anchoring member, where a straightener maintains the anchoring member in a compressed position. The components in the drawings are not necessarily to scale, emphasis instead being placed upon illustrating the principles of the teachings herein.

DETAILED DESCRIPTION OF THE DRAWINGS AND THE PRESENTLY PREFERRED EMBODIMENTS

Unless defined otherwise, all technical and scientific terms used herein have the same meaning as commonly understood by one of ordinary skill in the art to which this invention pertains.

The term "prosthesis" means any device for insertion or implantation into, or replacement, for a body part or function of that body part. It may also mean a device that enhances or adds functionality to a physiological system. The term prosthesis may include, for example and without limitation, a stent, stent-graft, filter, valve, balloon, embolization coil, and the like.

The term "endoluminal" refers to or describes the internal or inside of a lumen, duct, and other passageways or cavities located in a human or other animal body. A lumen or a body passageway may be an existing lumen or a lumen created by surgical intervention. As used in this specification, the terms "lumen" or "body passageway," and "vessel" are intended to have a broad meaning and encompass any duct (e.g., natural or iatrogenic) or cavity within the human body and may include, without limitation, blood vessels, respiratory ducts, gastrointestinal ducts, such as the biliary duct, intestines, the esophagus, the pericardial cavity, the thoracic cavity, and the like. Accordingly, the terms "endoluminal device" or "endoluminal prosthesis" describe devices that can be placed inside or moved through any such lumen or duct.

The terms "patient," "subject," and "recipient" as used in this application may refer to any animal, particularly humans.

The terms "proximal" and "distal" will be used to describe opposing axial ends of the ureteral stent, as well as the axial ends of various component features. The term "proximal" is used to refer to the end of the ureteral stent (or component thereof) that is closest to the operator during use of the system. The term "distal" is used to refer to the end of the ureteral stent (or component thereof) that is initially inserted into the patient, or that is closest to the patient during use.

The term "biocompatible" refers to a material that is substantially non-toxic in the in vivo environment of its intended use, and that is not substantially rejected by the patient's physiological system (i.e., is non-antigenic). This can be gauged by the ability of a material to pass the biocompatibility tests set forth in International Standards Organization (ISO) Standard No. 10993 and/or the U.S. Pharmacopeia (USP) 23 and/or the U.S. Food and Drug Administration (FDA) blue book memorandum No. G95-1, entitled "Use of International Standard ISO-10993, Biological Evaluation of Medical Devices Part-1: Evaluation and Testing."

Typically, these tests measure a material's toxicity, infectivity, pyrogenicity, irritation potential, reactivity, hemolytic activity, carcinogenicity and/or immunogenicity. A biocompatible structure or material, when introduced into a majority of patients, will not cause a significantly adverse, long-lived or escalating biological reaction or response, and is distinguished from a mild, transient inflammation which typically accompanies surgery or implantation of foreign objects into a living organism.

The term "medical device" means any object that is itself or that includes a component that is intentionally inserted into the body of a patient as part of a medical treatment, and that comprises a structure adapted for introduction into a patient. The medical device can be a tool, such as, without limitation, a catheter, a wire guide, a forceps, or a scissors used to affect a surgical procedure at and/or deliver a second medical device to a treatment site in a patient. An alternative medical device of the present invention is one that is commonly intended to be a permanent implant, such as a stent.

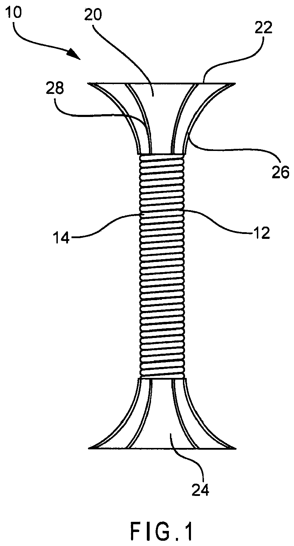

FIG. 1 depicts an embodiment of a prosthesis 10 of the present invention. The prosthesis 10 comprises a ureteral stent 12 having an elongated portion 12 having a proximal end 24 and a distal end 22 and a pair of anchoring members 20 disposed at each end. The elongated portion 14 of the stent 12 is configured to extend throughout the length of a patient's ureter. The elongated portion of the stent 10 may comprise a coiled wire 16. The coils 16 should be closely spaced so that they touch, but still allow fluid, such as urine or bile, to flow through the coils 16. The coils 16 should also be spaced closely enough so that no tissue ingrowth occurs. The coils 16 of the ureteral stent 12 provide the stent 12 with increased radial strength and help to maintain the lumen within the ureter and help to allow for drainage of urine through the ureter. Alternative embodiments of the ureteral stent 12 may comprise other configurations, including substantially tubular or helical. The stent 12 is typically available in sizes of about 1.67 mm to 2.67 mm (5 Fr to 8 Fr) having an inner diameter ranging from 1.1 mm to 1.78 mm. The stent 12 may be placed into the ureter using a guide wire and the procedure described below.

Materials used in the stents 12 are preferably biocompatible and corrosion-resistant. The coiled wire 16 is preferably made from alloys with minimal or low magnetic properties to avoid interference with diagnostic equipment, such as MRI machines. Alloys such as MP35N, MP 159, Astroloy M, Inconel 625, 316 stainless steel, 35N LT, Biodur 108, pure titanium, and Hastelloy S are preferred.

It should be understood that embodiments of the ureteral stent 10 may be manufactured from other biocompatible materials, including, but not limited to, polyester-based biocompatible polymers, nylon-based polymers, polytetrafluoroethylene (PTFE) polymers, silicone polymers, polyurethane polymers, polyethylene polymers, and thermoplastic polymers.

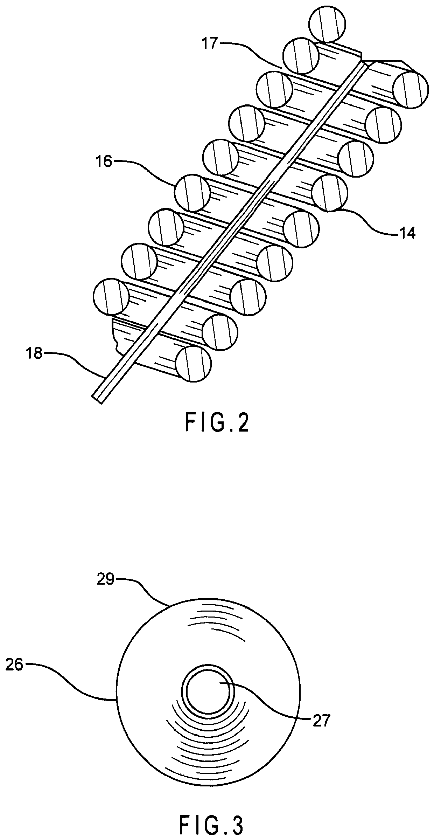

FIG. 2 shows the elongated portion 14 of the ureteral stent 12 in more detail.

The coils 16 have small gaps 17 between them so that urine may soak or leak into the stent in the kidney area or anywhere along the ureter and may leak out of the coils in the ureter or bladder area. The elongated portion 14 of the stent 12 between the anchoring members 20 is preferably about 20 cm to about 32 cm long. Other lengths may be used. The ureteral stent 12 may also include an inner wire 18 that extends throughout the length of the elongated portion 14. The wire 18 is preferably made from the same metallic alloy as the coil 16. The internal wire 18 is helpful in preventing unraveling or extension of the coils, especially when the stent is being removed. The internal wire 18 terminates at the distal and proximal ends 22, 24 of the ureteral stent 12.

Referring back to FIG. 1, the prosthesis 10 includes an anchoring member 20 positioned at the distal end 22 and the proximal end 24 of the stent 12. The anchoring member 20 on the distal end 22 of the ureteral stent 12 is positioned within the kidney in close proximity with an opening of the ureter. The anchoring member 20 on the proximal end 24 of the ureteral stent 12 is positioned within the bladder proximal to an opening of the ureter. The anchoring members 20 are designed to prevent migration of the stent 12 in both the distal and proximal directions. The anchoring members are capable of withstanding forces ranging from 0.3 N to 2.0 N. Each anchoring member 20 includes a wall 26 and a plurality of support struts 28. As shown, the wall 26 of the anchoring member 20 curves outward from the end of the elongated portion 14 of the stent 12 and has a generally concave configuration. Particularly, the diameter of the wall 26 increases as it curves outwardly. In some aspects, the shape of the wall 26 of the anchoring member 20 may be symmetrical about a central axis, as shown in FIG. 1. In other aspects, the anchoring member 20 may have an off-center configuration with respect to the elongated portion 14 of the stent 12. Preferably, the wall 26 of the anchoring member 20 is pliable and capable of being collapsed into a reduced diameter. In this embodiment, the anchoring members 20 have the same configuration. In other embodiments, the prosthesis 10 may include anchoring members 20 having different configurations at opposite ends of the ureteral stent 12. One exemplary embodiment includes an anchoring member 20 with a generally concave wall 26 having a plurality of support struts 28 at the distal end 22 of the stent 12 and a anchoring member 20 having a pigtail configuration at the proximal end 24 of the stent 12.

Suitable materials for the wall 26 of the anchoring member 20 may be selected from biocompatible materials. Examples of biocompatible materials from which the wall of the ureteral stent can be formed include, without limitation, polyesters, such as polyethylene terephthalate; fluorinated polymers, such as PTFE and expanded PTFE, and polyurethanes. For example, the wall 26 may be constructed from polyester, for example and without limitation, Dacron™, produced by DuPont. Dacron™ is known to be sufficiently biologically inert, non-biodegradable, and durable to permit safe insertion inside the human body.

The anchoring member 20 includes a plurality of support struts 28. The support struts 28 may be integral with the wall 26 of the anchoring member 20 or, alternatively, separately attached to the wall 26. The support struts 28 may either be positioned on an outer surface of the wall 26 of the anchoring member 20, or within the inner surface of the wall 26 of the anchoring member 20. In the defined invention, the support struts 28 are disposed longitudinally about the circumference of the wall 26 of the anchoring member 20. In other examples not forming part of the invention, the support struts 28 may have other configurations including, but not limited to, circumferentially disposed about the surface of the wall 26 and helically disposed about the surface of the wall 26. The plurality of support struts 28 are configured to maintain the patency of the anchoring member 20 when the distal and proximal ends 22, 24 of the ureteral stent 12 are deployed within the ureter and the bladder of the patient, respectively, and reinforce the wall 26 of the anchoring member 20.

The support struts 28 may be manufactured from numerous metals and alloys. In one example, the support struts comprise a shape-memory material such as a nickel-titanium alloy ("Nitinol"). In another example, the support struts 28 comprise metal alloy, such as stainless steel. Moreover, the structure of the support struts 28 may be exemplary formed in a variety of ways to provide a suitable support structure. For example, one or more of the support struts 28 may be made from a woven wire structure, a laser-cut cannula, individual interconnected rings, or another pattern or design. While one exemplary arrangement is shown in FIG. 1, it will be appreciated that the exact number of support struts 28, and their location, may be varied. The resilience of the support struts 28 and the pliability of the anchoring member 20 allows the device diameter to be reduced to less than or equal to the diameter of the elongated portion of the stent 12. This feature allows the prosthesis 10 to be delivered using devices having lower profiles, which minimizes trauma to the patient.

FIG. 3 provides a top view of the anchoring member 20. The wall 26 of the anchoring member 20 has a first opening 27 having a first diameter and a second opening 29 having a second diameter. The first opening 27 of the anchoring member 20 is in fluid communication with the lumen of the stent 12. The diameter of the first opening 27 is substantially the same as the inner diameter of the elongated portion 14 of the stent 12. The second diameter of the second opening is greater than the first diameter of the first opening 27. The second opening 29 of the wall 26 may have a diameter of up to about 2 cm. The configuration of the wall 26 provides a drainage opening that has a greater cross-sectional area than a stent having side drainage ports. The available cross sectional area has a direct impact on flow rate through the stent 12. A reduction in available cross sectional area may diminish the ability of the stent 12 to drain sufficiently.

Table 1 shows the percentage of reduction of side-port cross sectional area for a 5 Fr stent and an 8 Fr stent with side drainage ports having a diameter of 0.8 mm and a side drainage port of 1.25 mm, respectively, as a result of an increase in encrustation thickness. Table 2 shows the percentage of reduction of cross sectional area for a 1.67 mm (5 Fr) stent and an 2.67 mm (8 Fr) stent, each stent having an anchoring member with a first opening having a diameter of 1.1 mm and 1.78 mm, respectively, as a result of an increase in encrustation thickness. The percentage of reduction of cross sectional area was calculated using the following formula:

$$\frac{CSA - \left(\frac{D - 2E}{2}\right)^2 \times \pi}{CSA} \times 100$$

where CSA is the cross sectional area, D is the diameter, and E is the thickness of encrustation. These calculations assume a uniform encrustation thickness over the entire surface of the stent.

**Table 1**

| | | Reduction of CSA of Sideport | | | | | |
|---|---|---|---|---|---|---|---|
| | | Encrustation Thickness (mm) | | | | | |
| Stent Size | Diameter (mm) | 0.1 | 0.2 | 0.3 | 0.4 | 0.5 | 0.6 |
| 5 Fr | 0.8 | 43.75 | 75.00 | 93.75 | 100.00 | N/A | N/A |
| 8 Fr | 1.25 | 29.44 | 53.76 | 72.96 | 87.04 | 96.00 | 99.84 |

**Table 2**

| Stent Size | Diameter (mm) | Reduction of CSA of Working Length ID | | | | | |
|---|---|---|---|---|---|---|---|
| | | Encrustation Thickness (mm) | | | | | |
| | | 0.1 | 0.2 | 0.3 | 0.4 | 0.5 | 0.6 |
| 5 Fr | 1.1 | 33.06 | 59.50 | 79.34 | 92.56 | 99.17 | N/A |
| 8 Fr | 1.78 | 21.21 | 39.89 | 56.05 | 69.69 | 80.80 | 89.38 |

As shown, the effect of an identical amount of encrustation on the flow rate through the first opening of the anchoring member is reduced as compared to a similarly sized stent having side drainage ports. The first opening of the anchoring member of the ureteral stent provides a greater cross-sectional area for the drainage of urine as the thickness of encrustation increases. As a result, the first opening of the anchoring member of the ureteral stent minimizes the effects of encrustation on the stent over time. This provides a great benefit to a patient, as the ureteral stent could be used for a longer period of time. Furthermore, the improved drainage characteristics of the ureteral stent reduce the need for repeated procedures to remedy problems with blockage, which can cause added trauma to the patient.

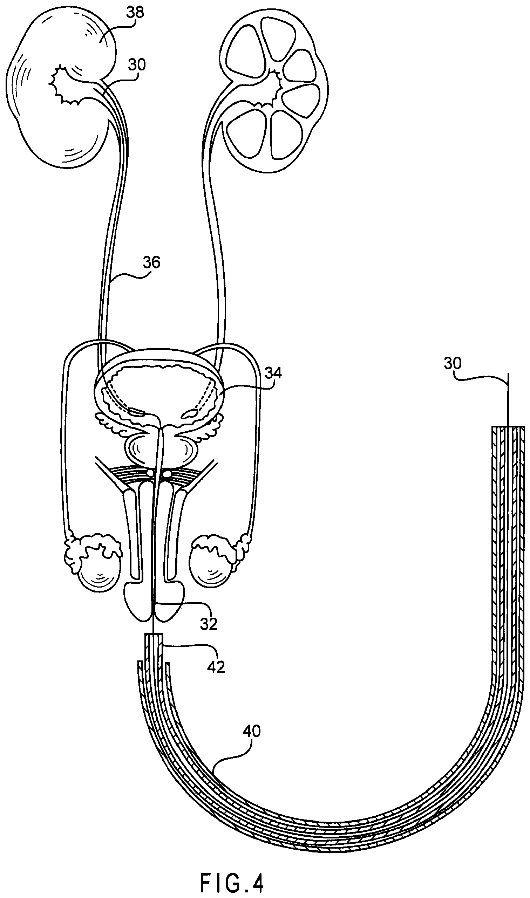

A method of introducing the prosthesis 10 is shown in FIG. 4. In this method, a physician places a wire guide 30 through a urethra 32, a bladder 34, and a ureter 36 into a kidney 38. After the wire guide 30 is placed, a catheter 42 secured to an access sheath 40 is guided along the wire guide 30, the catheter 42 and access sheath 40 combination coaxially "dual dilating" at least the proximal portion of the ureter 36. This coaxial dilatation procedure enables the physician to use a shorter wire guide, e.g., using a 145 or 150 cm wire guide rather than a wire guide that may have to be 220 cm or even longer, perhaps 250-260 cm. This may also shorten the time required to position the access sheath 40, and thus shorten the actual time spent in the therapeutic procedure and reduce the number of personnel required.

The distal opening of the access sheath 40 is generally positioned at or near the ureteropelvic junction of the patient. The prosthesis 10 is inserted into the access sheath 40 and the catheter 42 is used to advance the prosthesis 10 through the inner lumen of the access sheath 40. The catheter 42 is advanced through the access sheath 40 until the distal end 22 of the prosthesis 10 exits the distal opening of the access sheath 40, which results in an expansion of the distal anchoring member 20 into the ureteropelvic junction of the kidney. The position of the catheter 42 is maintained by the physician while withdrawing the access sheath 40. This continual withdrawal of the access sheath 40 allows the proximal anchoring member 20 to be expanded and deployed in the bladder of the patient.

Following the deployment of the proximal anchoring member 20, the physician can remove the catheter 42 and the access sheath 40 from the patient.

In addition to the method shown in FIG. 4, there are other ways to practice the invention. For instance, rather than accessing the ureter through the urethra and bladder, a physician may use a nephrostomy method, in which the access sheath and catheter are advanced through a person's skin to reach the calices of the kidney directly. If a path to a bile duct is needed, the physician may access the bile duct through an endoscope via the mouth, esophagus, stomach and intestines, or via laparoscopic methods directly through the skin (percutaneous).

If vascular access is desired, a physician may access the blood vessel through an opening, such as an opening manufactured in the femoral artery.

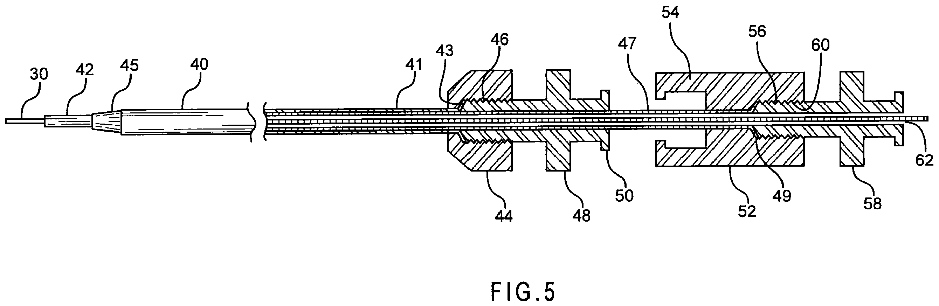

An example of a kit useful in the above procedure is depicted in FIG. 5. The kit includes a wire guide 30, which may be shorter than a wire guide used for a sequential procedure as described above. A wire guide 30 with a length of about 145-150 cm is preferred, but other lengths may be used. A catheter 42 is included, the catheter 42 preferably having a proximal end 47 with a flared tip 49.

Materials for the catheter are typically plastic or elastomeric materials, e.g., PVC, PTFE, polyurethane, silicone, and urethane, but any medically acceptable materials may be used. Catheters 42 suitable for this use are preferably about 50-85 cm long. The tip 49 is flared for ease in securing to connectors and in sealing with connectors so that the catheter may deliver a fluid, such as a radiopaque fluid for diagnostic procedures or for visualization purposes. The catheter 42 may have a hydrophilic coating on at least part of its outer surface.

Catheter 42 may interface with one or more connectors 52 for mating with syringe adapter 58 (such as a female Luer lock adapter) so that a syringe (not shown) can inject the radiopaque fluid. Connector 52 may include a male Luer lock fitting 54 on a distal end of connector 52 and internal threads 56 on its proximal end. Male Luer lock connection 54 may be used to connect first connector 52 to second connector 44. Threads 56 may interface with matching external threads 60 of a syringe adapted for delivery of a fluid through lumen 62. Flared end 49 of the catheter 42 helps to seal the connection between connector 52, catheter 42, and syringe adapter 58. While the Luer lock and threaded connections depicted and described are preferred, other connectors may be used instead. For example, quick-release connectors could be used to secure the catheter or sheath to their proximal fittings. When connectors 52 and 58 are joined with flared end 49, a leak-tight connection is formed, and the catheter may reliably deliver fluid without undesirable leakage.

Access sheath 40 includes a proximal portion 41 and an end portion with a flared tip 43. The access sheath also includes a distal end 45. The distal end 45 may be atraumatic, soft and rounded or tapered for ease of introduction into the patient. Distal end 45 of the access sheath 40 is also preferably more highly radiopaque than the remainder of the access sheath, so that the end may be observed with x-ray or fluoroscopic detection device during the implantation procedure. Flared tip 43 helps to seal an interface between access sheath 40 and connector 44. Access sheaths are preferably made from low friction polymers (e.g. PTFE, FEP etc.) with reasonable radial compressive strength-wire reinforcement added to the sheath for extra radial strength. Suitable access sheaths sold under the name of Check-Flo® II Introducer sheaths sold by Cook Incorporated, Bloomington, Ind. may be used. Also Flexor® sheaths available from Cook Urological Incorporated of Spencer, Ind. may be used. In this application the sheath is typically 70 cm long so to extend from the ureteral meatus to the ureteropelvic junction. The access sheath is generally just slightly larger in inner diameter than the outer diameter of the catheter. Connector 44 may include internal threads 46 for connecting to Luer lock connector 48 having female Luer lock connection 50. While Luer lock connections and connectors are preferred, other types of medically acceptable connectors may be used. At least a distal portion of sheath 40 may also include a hydrophilic coating.

The fittings described above may be used to connect access sheath 40 with catheter 42. To help insure that access sheath 40 seals securely, connector 44 may be temporarily joined to connector 48 with an adhesive. Other methods may also be used, such as securely tightening connectors 44, 48 together. Joining the female Luer lock connection 50 to male Luer lock connection 54 reliably secures access sheath 40 to catheter 42 for insertion or for removal. By breaking the connection between connectors 48, 52 after insertion, catheter 42 may be removed and the access sheath 40 may be used for other purposes. These other purposes may include diagnostic purposes, such as insertion of an endoscope, or therapeutic procedures, such as breaking up stones or calculi, using a holmium laser or other type of lithotripter. A grasper or basket may then be inserted into the working channel of the endoscope to remove the fragments.

In the same manner, connectors 52, 58 may also be temporarily joined with an adhesive to prevent easily breaking the connection. By adhering connector pairs 44, 48 and 52, 58, it is easier for the surgeon to make and break the Luer lock connection between connectors 48, 52.

In the assembled view of FIG. 5, note that the catheter 42 may be longer than the access sheath, and may extend slightly further distally than the access sheath 40. Nevertheless, the sheath and the catheter are substantially coaxial, i.e., catheter 42 runs the entire length of access sheath 40. Substantially coaxial device means that substantially the length of one of the sheath and the catheter is coaxial with the other of the sheath 40 and the catheter 42 during the procedure for implanting a stent or other device into a human or mammalian body.

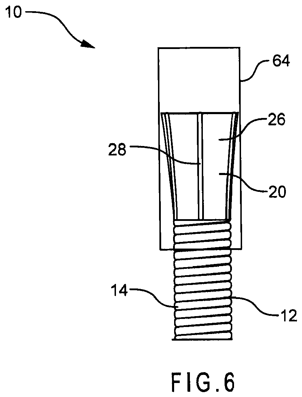

FIG. 6 depicts an urethral stent 12 having an anchoring member 20 maintained in a compressed position prior to deployment into a patient. As shown, a straightener 64 may be used to compress the support struts 28 and collapse the wall 26 of the anchoring member 20. The straightener 64 allows the anchoring member 20 to be reduced to the diameter of the elongated portion 14 of the stent 12. This lower profile allows the stent 12 to be delivered to the target location through the access sheath 40.

Throughout this specification various indications have been given as to preferred and alternative embodiments of the invention. It should be understood that it is the appended claims, including all equivalents, that are intended to define the spirit and scope of this invention.

## Claims

1.   A ureteral stent (12) for placing in a body passage of a patient, comprising
an elongated portion (14) having a proximal end (24) and a distal end (22), the elongated portion defining a lumen therethrough; and
a distal anchoring member (20) on the distal end of the elongated portion;
wherein the distal anchoring member comprises a wall (26) curving outward from the distal end of the elongated portion,
and wherein the wall of the distal anchoring member includes a plurality of support struts (28) disposed longitudinally about a circumference of the wall of the distal anchoring member.

2.   The ureteral stent (12) of claim 1, comprising a proximal anchoring member on the proximal end of the elongated portion, the proximal anchoring member comprising a wall curving outward from the proximal end of the elongated portion, where the wall of the proximal anchoring member includes a plurality of support struts disposed longitudinally about the circumference of the wall of the proximal anchoring member.

3.   The ureteral stent (12) of any preceding claim, where the support struts (28) are comprised of a shape memory material.

4. The ureteral stent (12) of any preceding claim, where at least one anchoring member (20) is symmetrical about a central longitudinal axis.

5. The ureteral stent (12) of any preceding claim, where the wall (26) of each anchoring member (20) includes a first opening (27) and a second opening (29).

6. The ureteral stent (12) of claim 5, where the first opening (27) has a first diameter and the second opening (29) has a second diameter greater than the first diameter.

7. The ureteral stent (12) of any preceding claim, where at least one anchoring member (20) is comprised of material selected from the group consisting of polyesters, fluorinated polymers, and polyurethanes.

8. The ureteral stent (12) of any preceding claim, where the elongated portion (14) comprises a coiled wire (16).

9. The ureteral stent (12) of claim 8, where the wall (26) of the anchoring means is comprised of a material different from the coiled wire (16) of the elongated portion (14).

10. The ureteral stent (12) of claims 1 to 7, wherein the elongated portion (14) comprises a coiled wire (16), the wire having an internal lumen, the internal lumen communicating outside the coiled wire through small spaces (17) between adjacent coils.

11. The ureteral stent (12) of any preceding claim, where the plurality of support struts (28) are integral with the outer surface of the wall (26).

12. The ureteral stent (12) of claim 10, where the wire is selected from the group consisting of MP35N, MP159, Astroloy M, Inconel 625, 315 stainless steel, 35N LT, Bidur 108, titanium, and Hastelloy S.

13. An anchoring member (20) for a ureteral stent (12), the anchoring member, comprising:

a wall (26) comprising a first opening (27) having a first diameter and a second opening having a second diameter defining an edge, and
a plurality of support struts (28) disposed about an outer surface of the wall said support struts disposed longitudinally about a circumference of the wall,

where the second diameter of the second opening of the wall is greater than the first diameter of the first opening of the wall and wherein the wall curves outwards from the first opening to the second opening.

14. The anchoring member (20) of claim 13, where the plurality of support struts (28) are disposed longitudinally about the circumference of the wall (26).

15. The anchoring member of claim 13, where the plurality of support struts (28) are integral with the outer surface of the wall (26).


**Patentansprüche**

1. Harnleiterstent (12) zum Platzieren in einem Körperdurchgang eines Patienten, umfassend:

einen länglichen Abschnitt (14) mit einem proximalen Ende (24) und einem distalen Ende (22), wobei der längliche Abschnitt ein hindurchführendes Lumen definiert; und
ein distales Verankerungselement (20) an dem distalen Ende des länglichen Abschnitts;
wobei das distale Verankerungselement eine Wand (26) umfasst, die von dem distalen Ende des länglichen Abschnitts auswärts gekrümmt ist,
und wobei die Wand des distalen Verankerungselements eine Vielzahl von Trägerstreben (28) einschließt, die in Längsrichtung um einen Umfang der Wand des distalen Verankerungselements herum angeordnet sind.

**2.** Harnleiterstent (12) nach Anspruch 1, umfassend ein proximales Verankerungselement an dem proximalen Ende des länglichen Abschnitts, wobei das proximale Verankerungselement eine Wand umfasst, die von dem proximalen Ende des länglichen Abschnitts auswärts gekrümmt ist, wobei die Wand des proximalen Verankerungselements eine Vielzahl von Trägerstreben einschließt, die in Längsrichtung um den Umfang der Wand des proximalen Verankerungselements herum angeordnet sind.

**3.** Harnleiterstent (12) nach einem der vorhergehenden Ansprüche, wobei die Trägerstreben (28) aus einem Formgedächtnismaterial zusammengesetzt sind.

**4.** Harnleiterstent (12) nach einem der vorhergehenden Ansprüche, wobei mindestens ein Verankerungselement (20) um eine zentrale Längsachse herum symmetrisch ist.

**5.** Harnleiterstent (12) nach einem der vorhergehenden Ansprüche, wobei die Wand (26) jedes Verankerungselements (20) eine erste Öffnung (27) und eine zweite Öffnung (29) einschließt.

**6.** Harnleiterstent (12) nach Anspruch 5, wobei die erste Öffnung (27) einen ersten Durchmesser aufweist und die zweite Öffnung (29) einen zweiten Durchmesser aufweist, der größer als der erste Durchmesser ist.

**7.** Harnleiterstent (12) nach einem der vorhergehenden Ansprüche, wobei mindestens ein Verankerungselement (20) aus einem Material ausgewählt aus der Gruppe bestehend aus Polyestern, fluorierten Polymeren sowie Polyurethanen zusammengesetzt ist.

**8.** Harnleiterstent (12) nach einem der vorhergehenden Ansprüche, wobei der längliche Abschnitt (14) einen gewickelten Draht (16) umfasst.

**9.** Harnleiterstent (12) nach Anspruch 8, wobei die Wand (26) des Verankerungsmittels aus einem Material zusammengesetzt ist, das sich von dem gewickelten Draht (16) des länglichen Abschnitts (14) unterscheidet.

**10.** Harnleiterstent (12) nach den Ansprüchen 1 bis 7, wobei der längliche Abschnitt (14) einen gewickelten Draht (16) umfasst, wobei der Draht ein Innenlumen aufweist, wobei das Innenlumen mit der Außenseite des gewickelten Drahtes durch kleine Räume (17) zwischen benachbarten Wicklungen kommuniziert.

**11.** Harnleiterstent (12) nach einem der vorhergehenden Ansprüche, wobei die Vielzahl der Trägerstreben (28) integral mit der Außenfläche der Wand (26) sind.

**12.** Harnleiterstent (12) nach Anspruch 10, wobei der Draht ausgewählt ist aus der Gruppe bestehend aus MP35N, MP159, Astroloy M, Inconel 625, rostfreiem Stahl 315, 35N LT, Bidur 108, Titan und Hastelloy S.

**13.** Verankerungselement (20) für einen Harnleiterstent (12), wobei das Verankerungselement umfasst:

eine Wand (26), umfassend eine erste Öffnung (27) mit einem ersten Durchmesser und eine zweite Öffnung mit einem zweiten Durchmesser, wodurch eine Kante definiert wird, und
eine Vielzahl von Trägerstreben (28), die um eine Außenfläche der Wand herum angeordnet sind, wobei die Trägerstreben in Längsrichtung um einen Umfang der Wand herum angeordnet sind,
wobei der zweite Durchmesser der zweiten Öffnung der Wand größer als der erste Durchmesser der ersten Öffnung der Wand ist, und wobei die Wand von der ersten Öffnung zu der zweiten Öffnung auswärts gekrümmt ist.

**14.** Verankerungselement (20) nach Anspruch 13, wobei die Vielzahl der Trägerstreben (28) in Längsrichtung um den Umfang der Wand (26) herum angeordnet ist.

**15.** Verankerungselement nach Anspruch 13, wobei die Vielzahl der Trägerstreben (28) integral mit der Außenfläche der Wand (26) ist.

**Revendications**

**1.** Stent urétéral (12) destiné à être placé dans un passage corporel d'un patient, comprenant :

une partie allongée (14) comportant une extrémité proximale (24) et une extrémité distale (22), la partie allongée définissant une lumière la traversant ; et

un élément de fixation distal (20) sur l'extrémité distale de la partie allongée ;

l'élément de fixation distal comprenant une paroi (26) s'incurvant vers l'extérieur à partir de l'extrémité distale de la partie allongée,

et la paroi de l'élément de fixation distal comprenant une pluralité d'éléments structurels de support (28) disposés longitudinalement autour d'une circonférence de la paroi de l'élément de fixation distal.

2. Stent urétéral (12) selon la revendication 1, comprenant un élément de fixation proximal sur l'extrémité proximale de la partie allongée, l'élément de fixation proximal comprenant une paroi s'incurvant vers l'extérieur à partir de l'extrémité proximale de la partie allongée, la paroi de l'élément de fixation proximal comprenant une pluralité d'éléments structurels de support disposés longitudinalement autour de la circonférence de la paroi de l'élément de fixation proximal.

3. Stent urétéral (12) selon l'une quelconque des revendications précédentes, dans lequel les éléments structurels de support (28) sont composés d'un matériau à mémoire de forme.

4. Stent urétéral (12) selon l'une quelconque des revendications précédentes, dans lequel au moins un élément de fixation (20) est symétrique autour d'un axe longitudinal central.

5. Stent urétéral (12) selon l'une quelconque des revendications précédentes, dans lequel la paroi (26) de chaque élément de fixation (20) comprend une première ouverture (27) et une seconde ouverture (29).

6. Stent urétéral (12) selon la revendication 5, dans lequel la première ouverture (27) présente un premier diamètre et la seconde ouverture (29) présente un second diamètre supérieur au premier diamètre.

7. Stent urétéral (12) selon l'une quelconque des revendications précédentes, dans lequel au moins un élément de fixation (20) est composé d'un matériau sélectionné dans le groupe constitué de polyesters, de polymères fluorés et de polyuréthanes.

8. Stent urétéral (12) selon l'une quelconque des revendications précédentes, dans lequel la partie allongée (14) comprend un fil spiralé (16).

9. Stent urétéral (12) selon la revendication 8, dans lequel la paroi (26) des moyens de fixation est composée d'un matériau différent de celui du fil spiralé (16) de la partie allongée (14).

10. Stent urétéral (12) selon les revendications 1 à 7, dans lequel la partie allongée (14) comprend un fil spiralé (16), le fil comportant une lumière intérieure, la lumière intérieure communiquant avec l'extérieur du fil spiralé par de petits espaces (17) entre des spires adjacentes.

11. Stent urétéral (12) selon l'une quelconque des revendications précédentes, dans lequel la pluralité d'éléments structurels de support (28) font partie intégrante de la surface extérieure de la paroi (26).

12. Stent urétéral (12) selon la revendication 10, dans lequel le fil est sélectionné dans le groupe constitué du MP35N, du MP159, de l'Astroloy M, de l'Inconel 625, de l'acier inoxydable 315, du 35N LT, du Bidur 108, du titane et de l'Hastelloy S.

13. Élément de fixation (20) pour un stent urétéral (12), l'élément de fixation comprenant :

une paroi (26) comprenant une première ouverture (27) présentant un premier diamètre et une seconde ouverture présentant un second diamètre définissant un bord, et

une pluralité d'éléments structurels de support (28) disposés autour d'une surface extérieure de la paroi, lesdits éléments structurels de support étant disposés longitudinalement autour d'une circonférence de la paroi,

dans lequel le second diamètre de la seconde ouverture de la paroi est supérieur au premier diamètre de la première ouverture de la paroi et dans lequel la paroi s'incurve vers l'extérieur depuis la première ouverture vers la seconde ouverture.

14. Élément de fixation (20) selon la revendication 13, dans lequel la pluralité d'éléments structurels de support (28) sont disposés longitudinalement autour de la circonférence de la paroi (26).

**15.** Élément de fixation selon la revendication 13, dans lequel la pluralité d'éléments structurels de support (28) font partie intégrante de la surface extérieure de la paroi (26).

FIG.1

FIG.2

FIG.3

FIG.4

FIG.5

10

64

26

28

20

14

12

FIG.6

**EP 2 609 892 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0191668 A
- US 20040087886 A